# EUROPEAN PATENT APPLICATION

(11) **EP 4 563 639 A1**
(43) Date of publication of application: **04.06.2025**
(21) Application number: 24196416.2
(22) Date of filing: 26.08.2024
(51) Int. Cl.: C08L 1/28, A61K 8/02, A61K 31/717

(54) **POROUS CELLULOSE PARTICLES AND COSMETIC PREPARATION**

(30) Priority: 28.11.2023 JP 2023200928; 27.03.2024 JP 2024052554
(71) Applicant: Fujifilm Business Innovation Corp., Tokyo 107-0052 (JP)
(72) Inventor: MATOBA, Shota, Minamiashigara (JP); OKI, Masahiro, Minamiashigara (JP); ISHIZUKA, Takahiro, Minamiashigara (JP); YOSHIKAWA, Hideaki, Minamiashigara (JP); IWADATE, Yuko, Minamiashigara (JP); TAKEUCHI, Sakae, Minamiashigara (JP)
(74) Representative: Meissner Bolte Partnerschaft mbB

(57) **Abstract**

Porous cellulose particles contain a cellulose, a cellulose derivative, or a mixture thereof as a main component, in which the porous cellulose particles have open cells extending from a particle surface to a particle interior, and have a BET specific surface area of 1.7 m²/g or more and 24.0 m²/g or less.

## Description

### Background

### (i) Technical Field

The present disclosure relates to porous cellulose particles and a cosmetic preparation.

### (ii) Related Art

Japanese Patent No. 7269239 proposes porous cellulose particles formed by assemblies of type-I crystalline cellulose obtained by a process that does not involve intentional chemical modification, in which the crystalline cellulose has a type-I crystal form composed of glucose molecules as constitutional units, and the porous cellulose particles have an average particle size d1 of 0.5 µm or more and less than 50 µm, a specific surface area of 25 to 1000 m²/g, and a sphericity of 0.85 or more.

International Patent Publication No. 2018/147213 proposes cellulose particles having a volume-average particle size of 50 µm or more and 1000 µm or less and a linseed oil absorption of 150 mL/100 g or more.

### Summary

Accordingly, it is an object of the present disclosure to provide porous cellulose particles that contain a cellulose, a cellulose derivative, or a mixture thereof as a main component and that have open cells extending from a particle surface to a particle interior, according to which porous cellulose particles having excellent mechanical strength and excellent rolling property and oil absorbing property after application of mechanical load are provided compared to when the BET specific surface area is less than 1.7 m²/g or more than 24.0 m²/g.

According to a first aspect of the present disclosure, there is provided porous cellulose particles containing a cellulose, a cellulose derivative, or a mixture thereof as a main component, in which the porous cellulose particles have open cells extending from a particle surface to a particle interior, and have a BET specific surface area of 1.7 m²/g or more and 24.0 m²/g or less.

According to a second aspect of the present disclosure, there is provided the porous cellulose particles according to the first aspect, in which the BET specific surface area is 8.0 m²/g or more and 20.0 m²/g or less.

According to a third aspect of the present disclosure, there is provided the porous cellulose particles according to the first or second aspect, in which, when cross sections of the porous cellulose particles are observed, an area ratio of the open cells is 15% or more and 90% or less.

According to a fourth aspect of the present disclosure, there is provided the porous cellulose particles according to the third aspect, in which, when cross sections of the porous cellulose particles are observed, the area ratio of the open cells is 25% or more and 85% or less.

According to a fifth aspect of the present disclosure, there is provided the porous cellulose particles according to any one of the first to fourth aspects, in which the cellulose derivative is cellulose acylate having a degree of substitution of 0.6 or less.

According to a sixth aspect of the present disclosure, there is provided the porous cellulose particles according to the fifth aspect, in which the cellulose derivative is cellulose acylate having a degree of substitution of 0.2 or less.

According to a seventh aspect of the present disclosure, there is provided the porous cellulose particles according to any one of the first to sixth aspects, in which the porous cellulose particles have a volume-average particle size of 3 µm or more and 45 µm or less.

According to an eighth aspect of the present disclosure, there is provided a cosmetic preparation comprising the porous cellulose particles according to any one of the first to seventh aspects.

According to the first aspect of the present disclosure, there is provided porous cellulose particles that contain a cellulose, a cellulose derivative, or a mixture thereof as a main component and that have open cells extending from a particle surface to a particle interior, according to which porous cellulose particles having excellent mechanical strength and excellent rolling property and oil absorbing property after application of mechanical load are provided compared to when the BET specific surface area is less than 1.7 m²/g or more than 24.0 m²/g.

According to the second aspect of the present disclosure, there is provided porous cellulose particles that have excellent mechanical strength and excellent rolling property and oil absorbing property after application of mechanical load compared to when the BET specific surface area is less than 8.9 m²/g or more than 20.0 m²/g.

According to the third aspect of the present disclosure, there is provided porous cellulose particles that have excellent mechanical strength and excellent rolling property and oil absorbing property after application of mechanical load compared to when the area ratio of the open cells is less than 15% or more than 90%.

According to the fourth aspect of the present disclosure, there is provided porous cellulose particles that have excellent mechanical strength and excellent rolling property and oil absorbing property after application of mechanical load compared to when the area ratio of the open cells is less than 25% or more than 85%.

According to the fifth aspect of the present disclosure, there is provided porous cellulose particles that have excellent mechanical strength and excellent rolling property and oil absorbing property after application of mechanical load compared to when the cellulose derivative is cellulose acylate having a degree of substitution exceeding 0.6.

According to the sixth aspect of the present disclosure, there is provided porous cellulose particles that have excellent mechanical strength and excellent rolling property and oil absorbing property after application of mechanical load compared to when the cellulose derivative is cellulose acylate having a degree of substitution exceeding 0.2.

According to the seventh aspect of the present disclosure, there is provided porous cellulose particles that have excellent mechanical strength and excellent rolling property and oil absorbing property after application of mechanical load compared to when the porous cellulose particles have a volume-average particle size of less than 3 µm or more than 45 µm.

According to the eighth aspect of the present disclosure, there is provided a cosmetic preparation that has excellent mechanical strength and excellent smoothness and sebum adsorbing property after application of mechanical load compared to when porous cellulose particles that contain a cellulose, a cellulose derivative, or a mixture thereof as a main component, that have open cells extending from a particle surface to a particle interior, and that have a BET specific surface area of less than 1.7 m²/g or more than 24.0 m²/g are used.

### Detailed Description

One exemplary embodiment of the present disclosure will now be described. The following description and examples are merely exemplary embodiments and do not limit the scope of the disclosure.

In stepwise numerical ranges of the present description, the upper limit or the lower limit of one numerical range may be substituted with an upper limit or a lower limit of a different stepwise numerical range. In any numerical range recited in the present description, the upper limit or the lower limit of that numerical range may be substituted with any value disclosed in Examples.

Each of the components may contain multiple corresponding substances.

When the amount of a component in a composition is described and when there are two or more substances that correspond to that component in the composition, the amount is the total amount of the two or more substances in the composition unless otherwise noted.

### Cellulose particles

The porous cellulose particles according to an exemplary embodiment contain a cellulose, a cellulose derivative, or a mixture thereof as a main component.

The porous cellulose particles of the exemplary embodiment have open cells extending from a particle surface to a particle interior, and have a BET specific surface area of 1.7 m²/g or more and 24.0 m²/g or less.

The porous cellulose particles according to the exemplary embodiment have excellent mechanical strength due to the features described above, and the rolling property and the oil absorbing property of the porous cellulose particles after application of mechanical load are also excellent. The reason for this is presumably as follows.

A porous cellulose containing, as a main component, a cellulose, a cellulose derivative, or a mixture thereof has light touch, a high rolling property, and a high oil absorbing property.

However, the mechanical strength is low, and, for example, when a porous cellulose is used as an additive in a product such as a cosmetic preparation and when the porous cellulose is put under mechanical load such as pressing during manufacture of the product, the particles deform, the porous structure collapses, and the rolling property and the oil absorbing property become degraded.

In contrast, the porous cellulose particles of the exemplary embodiment, which have a porous structure having open cells that extend from a particle surface to a particle interior, exhibit enhanced rolling property and oil absorbing property. In addition, decreasing the BET specific surface area of the porous cellulose particles to 1.7 m²/g or more and 24.0 m²/g or less contributes to securing the volume of the open pores, enhancing the rolling property and the oil absorbing property, securing the thickness of the walls between the open cells, and increasing the mechanical strength.

It is assumed from the above-described features that the porous cellulose particles according to the exemplary embodiment exhibit excellent mechanical strength, and excellent rolling property and oil absorbing property after application of mechanical load.

In particular, since the cellulose particles according to the exemplary embodiment have excellent mechanical strength and excellent rolling property and oil absorbing property after application of mechanical load, when the cellulose particles are used in a cosmetic preparation, a cosmetic preparation that has a porous structure that does not easily collapse during the production process and has excellent mechanical strength and excellent rolling property and oil absorbing property after application of mechanical load is obtained.

The porous cellulose particles according to an exemplary embodiment will now be described in detail.

### Porous structure of porous cellulose particles

Porous cellulose particles according to an exemplary embodiment have open cells that extend from a particle surface to a particle interior.

Specifically, for example, when cross sections of the porous cellulose particles are observed, the area ratio of the open cells is preferably 15% or more and 90% or less, more preferably 25% or more and 85% or less, yet more preferably 30% or more and 70% or less, and particularly preferably 35% or more and 70% or less.

When the area ratio of the open cells is within this range, a sufficient volume of open cells is secured, and the rolling property and the oil absorbing property are increased.

Confirmation of open cells and the area ratio of the open cells are as follows.

Porous cellulose particles are embedded in an epoxy resin and cut with a diamond knife or the like to prepare a specimen having an observation surface, which is a surface that includes cross sections of the porous cellulose particles.

The prepared specimen is loaded onto a scanning electron microscope (SEM) to obtain a SEM image (accelerating voltage: 1.0 kV, magnification: 20,000x) of cross sections of the cellulose particles imaged with the SEM.

In the porous cellulose particle cross sections, the presence of open cells that extend from the particle surface to the particle interior is confirmed from the obtained SEM image.

First, the obtained SEM image is binarized by image analysis software (for example, ImageJ, WinROOF) with a threshold set to clearly distinguish voids from the epoxy resin constituting the background. Next, the area ratio of all cells relative to the entire cross section of the porous cellulose particle is determined. Meanwhile, the area ratio of closed cells confined in the wall relative to the entire cross section of the porous cellulose particle is determined. For the closed cells, the area ratio may be calculated without binarizing if the closed cells are directly identifiable. Here, the cells that do not appear to be open cells in the cross sectional observation are considered closed cells.

Next, the area ratio of the open cells is calculated from the equation: area ratio of open cells = area ratio of all cells - area ratio of closed cells.

This operation is performed ten times, and the obtained values are arithmetically averaged.

Here, cross sections of porous cellulose particles to be observed are selected from the cross sections of porous cellulose particles that have a cross sectional size equal to or more than 85% of the volume-average particle size of the porous cellulose particles. Here, the cross sectional size refers to the length of the longest straight line drawn between arbitrarily selected two points on the contour of the porous cellulose particle cross section (in other words, the long axis).

### BET specific surface area of porous cellulose particles

The porous cellulose particles of the exemplary embodiment have a BET specific surface area of 1.7 m²/g or more and 24.0 m²/g or less.

When the BET specific surface area is 1.7 m²/g or more, the cellulose particles take a porous structure, not a solid structure, and the rolling property and the oil absorbing property are improved.

When the BET specific surface area is 24.0 m²/g or less, the walls between the open cells become thick, and the mechanical strength is improved. Thus, when the porous cellulose particles are used in a product such as a cosmetic preparation, the porous cellulose particles do not deform and do not suffer the collapse of the porous structure under the mechanical load, such as stirring, applied during manufacture of the product, and the rolling property and the oil absorbing property are improved. In addition, even when the porous cellulose particles are used alone, the porous cellulose particles do not deform and do not suffer the collapse of the porous structure under the mechanical load, and the rolling property and the oil absorbing property are improved.

Thus, the BET specific surface area is adjusted to be within the aforementioned range. The BET specific surface area is preferably 8.0 m²/g or more and 20 m²/g or less and more preferably 10 m²/g or more and 16 m²/g or less.

The BET specific surface area of the porous cellulose particles is measured with a specific surface area analyzer "Macsorb HM model-1201" produced by MOUNTECH Co., Ltd.

Specifically, after subjecting 50 mg of particles to a vacuum/34°C/one day pretreatment and to a 30°C/120 min pretreatment, the BET specific surface area is determined by a BET multipoint method using a nitrogen gas having a purity of 99.99% or higher.

### Layer structure of porous cellulose particles

The porous cellulose particles according to the exemplary embodiment may be porous cellulose particles that do not have a coating layer. In other words, the porous cellulose particles according to the exemplary embodiment may be single layer-structure porous cellulose particles that contain a cellulose, a cellulose derivative, or a mixture thereof as a main component.

### Components of porous cellulose particles

The porous cellulose particles according to an exemplary embodiment contain a cellulose, a cellulose derivative, or a mixture thereof as a main component.

Here, the meaning of the phrase "contain a cellulose, a cellulose derivative, or a mixture thereof as a main component" is that the amount of the cellulose, the cellulose derivative, or the mixture thereof relative to the amount of the porous cellulose particles is 90 mass% or more (preferably 95 mass% or more, more preferably 98 mass% or more, and most preferably 100 mass%).

### Cellulose

The number-average molecular weight of cellulose is preferably 37000 or more and more preferably 45000 or more.

There is no upper limit for the number-average molecular weight of cellulose, and the upper limit may be, for example, 100,000 or less.

The number-average molecular weight of cellulose is measured by gel permeation chromatography (differential refractometer Optilab T-rEX produced by Wyatt Technology Corporation, a multi angle light scattering detector DAWN HELEOS II produced by Wyatt Technology Corporation, columns: TSKgel α-M and α-3000, one each, produced by TOSOH CORPORATION) with dimethylacetamide (0.1 M lithium chloride added) serving as a solvent.

### Cellulose derivative

Examples of the cellulose derivative include cellulose acylate, cellulose ether, hydroxyalkyl cellulose, and carboxymethyl cellulose.

Among these, cellulose acylate is preferable as the cellulose derivative. Cellulose acylate tends to increase the rolling property, the oil absorbing property, and the mechanical strength.

Cellulose acylate is a cellulose derivative obtained by substituting at least one of the hydroxy groups in cellulose with an acyl group (acylation). An acyl group is a group having a -CO-R^{AC} (R^{AC} represents a hydrogen atom or a hydrocarbon group) structure.

Cellulose acylate is, for example, a cellulose derivative represented by general formula (CA) below.

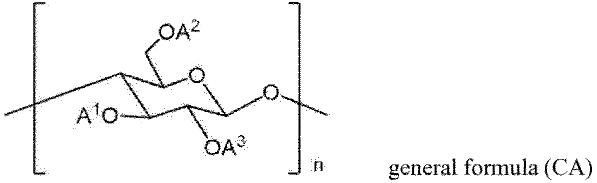

In general formula (CA), A¹, A², and A³ each independently represent a hydrogen atom or an acyl group, and n represents an integer of 2 or more. Here, at least one selected from n A¹, n A², and n A³ represents an acyl group In the molecule, n A¹ may be the same or partly the same, or different from one another. The same applies to n A² and n A³ in the molecule.

The acyl group represented by A¹, A², and A³ may have a linear, branched, or cyclic hydrocarbon group, preferably has a linear or branched hydrocarbon group, and more preferably has a linear hydrocarbon group.

The acyl group represented by A¹, A², and A³ may have a saturated or unsaturated hydrocarbon group and preferably has a saturated hydrocarbon group.

The acyl group represented by A¹, A², and A³ may have 1 or more and 6 or less carbon atoms. In other words, cellulose acylate preferably has an acyl group having 1 or more and 6 or less carbon atoms.

The acyl group represented by A¹, A², and A³ may have a hydrogen atom substituted with a halogen atom (for example, a fluorine atom, a bromine atom, or an iodine atom), an oxygen atom, or a nitrogen atom, for example, but is preferably unsubstituted.

Examples of the acyl group represented by A¹, A², and A³ include a formyl group, an acetyl group, a propionyl group, a butyryl group (butanoyl group), a propenoyl group, and a hexanoyl group. Among these, acyl groups having 2 or more and 4 or less carbon atoms is preferable and an acyl group having 2 or 3 carbon atoms is more preferable as the acyl group from the viewpoint of improving the biodegradation speed.

Examples of the cellulose acylate include cellulose acetate (cellulose monoacetate, cellulose diacetate (DAC), and cellulose triacetate), cellulose acetate propionate (CAP), and cellulose acetate butyrate (CAB).

From the viewpoint of improving the rolling property, the oil absorbing property, and the mechanical strength, cellulose acylate may be cellulose acetate.

One cellulose acylate may be used alone or two or more cellulose acylates may be used in combination.

The weight-average polymerization degree of cellulose acylate is preferably 200 or more and 1000 or less, more preferably 500 or more and 1000 or less, and yet more preferably 600 or more and 1000 or less.

The weight-average polymerization degree of cellulose acylate is determined by the following procedure from the weight-average molecular weight (Mw).

First, the weight-average molecular weight (Mw) of cellulose acylate is measured on a polystyrene basis using tetrahydrofuran with a gel permeation chromatograph (GPC analyzer HLC-8320GPC produced by TOSOH CORPORATION, column: TSKgel α-M).

Next, the result is divided by the molecular weight of the constitutional unit of the cellulose acylate to determine the polymerization degree of cellulose acylate. For example, when the substituent of the cellulose acylate is an acetyl group, the molecular weight of the constitutional unit is 263 when the degree of substitution is 2.4 and is 284 when the degree of substitution is 2.9.

From the viewpoint of improving the rolling property, the oil absorbing property, and the mechanical strength, the degree of substitution of cellulose acylate is preferably 0.75 or less, more preferably 0.6 or less, and yet more preferably 0.2 or less.

The degree of substitution of cellulose acylate is an indicator of the extent in which the hydroxy groups in the cellulose are substituted with acyl groups. In other words, the degree of substitution is an indicator of the extent of acylation of cellulose acylate. Specifically, the degree of substitution means an intramolecular average of how many of the three hydroxy groups in a D-glucopyranose unit of cellulose acylate are substituted with acyl groups. The degree of substitution is determined from an infrared absorption spectrum (Spotlight 400, produced by PerkinElmer Inc.) through the absorbance ratio between the cellulose-derived peak (1030 cm⁻¹) and the acyl group-derived peak (1738 cm⁻¹).

Specifically, the absorbance at 1738 cm⁻¹/absorbance at 1030 cm⁻¹ ratio is assumed to be the degree of substitution.

The degree of substitution of the cellulose acylate can be adjusted by the amount of the base (for example, sodium hydroxide) used in saponifying the cellulose acylate and the reaction time.

### Other components

The porous cellulose particles of this exemplary embodiment may contain other components.

Examples of the other components include plasticizers, flame retardants, compatibilizers, release agents, light stabilizers, weather stabilizers, coloring agents, pigments, modifiers, anti-drip agents, antistatic agents, anti-hydrolysis agents, fillers, reinforcing agents (for example, glass fibers, carbon fibers, talc, clay, mica, glass flakes, milled glass, glass beads, crystalline silica, alumina, silicon nitride, aluminum nitride, and boron nitride), acid acceptors for preventing acetic acid release (oxides such as magnesium oxide and aluminum oxide, metal hydroxides such as magnesium hydroxide, calcium hydroxide, aluminum hydroxide, and hydrotalcite, calcium carbonate, and talc), and reactive trapping agents (for example, epoxy compounds, acid anhydride compounds, and carbodiimide).

The amount of the other components contained relative to the total amount of the porous cellulose particles may be 0 mass% or more and 5 mass% or less for each component. Here, "0 mass%" means that other components are not contained.

### External additive

To the cellulose particles of the exemplary embodiment, inorganic particles may be added as an external additive. External addition of inorganic particles reduces secondary aggregation of the particles, and the particles easily exhibit their inherent properties. Thus, the rolling property, the oil absorbing property, and the mechanical strength tend to be improved.

The external additive is, for example, at least one selected from the group consisting of silicon-containing compound particles and metal oxide particles.

Silicon-containing compound particles refer to particles that contain silicon.

Silicon-containing compound particles may be particles solely composed of silicon or may be particles that contain silicon and other elements.

The silicon-containing compound particles may be silica particles. Silica particles may be any particles containing silica, that is, SiO₂, as a main component and may be crystalline or amorphous. The silica particles may be produced by using a silicon compound, such as water glass or alkoxysilane, as a raw material, or may be obtained by crushing quartz.

Oxides of metals other than silicon can be used as the metal oxide.

Examples of the metal oxide include zinc oxide, magnesium oxide, iron oxide, and aluminum oxide.

The volume-average particle diameter of the external additive is preferably 1 nm or more and 100 nm or less and more preferably 5 nm or more and 30 nm or less from the viewpoint of improving the rolling property, the oil absorbing property, and the mechanical strength.

The volume-average particle diameter of the external additive is measured by the same method as measuring the volume-average particle size of the cellulose.

The amount of the externally additive externally added relative to the mass of the entire cellulose particles (cellulose particles without external addition of the external additive) may be 0.1 mass% or more and 2 mass% or less.

### Volume-average particle size

The volume-average particle size of the cellulose particles according to the exemplary embodiment is preferably 1 µm or more and 100µm or less, more preferably 3 µm or more and 45 µm or less, and yet more preferably 4 µm or more and 15 µm or less.

By adjusting the volume-average particle size of the cellulose particles according to the exemplary embodiment to be 1 µm or more and 100 µm or less, the particles have an appropriate size, and the rolling property, the oil absorbing property, and the mechanical strength are improved.

The volume-average particle diameter of the cellulose particles is measured as follows.

Particle sizes are measured with an LS particle size distribution meter "Beckman Coulter LS 13 320 (produced by Beckman Coulter, Inc.)", the particle size cumulative distribution is plotted on a volume basis from the small diameter side, and the particle size at 50% is determined as the volume-average particle size.

### Method for producing porous cellulose particles

A method for producing cellulose particles may include a step of forming cellulose acylate particles (particle forming step) and a step of saponifying the cellulose acylate particles (saponifying step).

### Particle forming step

(1) First, cellulose acylate is added to a mixture of a water-soluble organic solvent A and a poor solvent, and the resulting mixture is heated to 50°C or higher and 70°C or lower to dissolve cellulose acylate in the water-soluble organic solvent A to prepare a cellulose acylate solution A.
(2) Next, the cellulose acylate solution A is added to a calcium carbonate dispersion containing calcium carbonate dispersed in water, and the resulting mixture is stirred while keeping the liquid temperature to 50°C or higher and 70°C or lower to prepare a cellulose acylate emulsion B. As a result of this operation, the poor solvent disperses in the cellulose acylate particles in the cellulose acylate emulsion B, and open cells are formed as a result.
(3) Next, the cellulose acylate emulsion B is added to a large quantity of hot water having a liquid temperature of 60°C or higher and 75°C or lower, and the resulting mixture is stirred all night to prepare a cellulose acylate particle dispersion C. As a result of operation, cellulose acylate particles are deposited while the open cells are maintained.
(4) Next, diluted hydrochloric acid is added to the cellulose acylate particle dispersion C to dissolve calcium carbonate, and then the residue is filtered.
(5) Next, the residue is dispersed in pure water to obtain a cellulose acylate particle dispersion D.

### Saponifying step

(6) Next, sodium hydroxide is added to the cellulose acylate particle dispersion D, and then the cellulose acylate particle dispersion D is heated in a weakly alkaline environment and stirred to saponify the cellulose acylate particles and prepare a cellulose particle suspension.

(7) Next, hydrochloric acid is added to the cellulose particle suspension to adjust the pH of the suspension to near neutral (for example, in the range of 6.5 or more and 7 or less), and then the cellulose particles are filtered out and washed with an organic solvent. Subsequently, the cellulose particles are repeatedly filtered out and washed with pure water. After the electrical conductivity of the filtrate has reached 10 µs/cm or less, the filtered out cellulose particles are dried.

Through the aforementioned steps, porous cellulose particles containing a cellulose as a main component are obtained.

Examples of the method for obtaining porous cellulose particles containing cellulose acetate as a main component include a method that involves obtaining cellulose acylate particles in the particle forming step without carrying out the saponifying step and a method involving decreasing the amount of sodium hydroxide in the saponifying step so as to decrease the extent of the saponification. The degree of substitution of cellulose acetate can be adjusted by decreasing the amount of sodium hydroxide in the saponifying step.

In order to obtain porous cellulose particles containing a cellulose derivative other than cellulose acetate as a main component, the saponifying step is omitted, and a cellulose derivative is used instead of cellulose acetate when performing the particle forming step to obtain cellulose derivative particles.

Here, the water-soluble organic solvent is a solvent in which 0.1 mass% or more and 10 mass% or less of water dissolves at 25°C relative to the solvent, and examples thereof include ethyl acetate and butyl acetate.

The poor solvent is a solvent in which less than 0.1 mass% of a cellulose derivative dissolves at 25°C relative to the solvent, and examples thereof include alcohols (heptanol, etc.) having a boiling point of 100°C or higher.

Examples of the organic solvent used in washing the cellulose particles include acetone and alcohols (methanol, ethanol, propanol, etc.).

### External addition step

An external additive may be added to the obtained cellulose particles.

In the external addition step, for example, an external additive is added to the cellulose particles by using a mixing mill, a V blender, a Henschel mixer, a Lodige mixer, or the like.

### Usage

Examples of the usage of the cellulose particles of the present exemplary embodiment include particles for cosmetics, rolling agents, abrasives, scrubbing agents, display spacers, bead molding materials, light diffusing particles, resin reinforcing agents, refractive index controllers, biodegradation accelerators, fertilizers, water-absorbing particles, toner particles, and antiblocking particles.

### Cosmetic preparation

A cosmetic preparation according to an exemplary embodiment contains the cellulose particles according to an exemplary embodiment.

Examples of the cosmetic preparation according to the exemplary embodiment include cosmetic preparations for base makeup (for example, makeup bases, concealers, foundations, and face powders), cosmetic preparations for makeups (for example, lipsticks, lip-glosses, lipliners, blushes, eyeshadows, eyeliners, mascaras, eyebrow pencils, nail manicures, and nail caring cosmetic items), and cosmetic preparations for skin care (for example, face wash formulations, face cleansers, lotions, milky lotions, serums, packs, facial masks, and cosmetic preparations for eye areas and mouth areas).

In particular, since the cosmetic preparation according to the exemplary embodiment is highly smooth and has a high sebum adsorbing property, the cosmetic preparation may be used for makeup.

### EXAMPLES

Examples, which do not limit the scope of the present disclosure, will now be described. In the description below, "parts" and "%" are all on a mass basis unless otherwise noted.

### Example 1

### Particle forming step

To 480 parts of a mixture of ethyl acetate and heptanol (acting as a poor solvent) (amount of poor solvent in the mixture: 20%), 120 parts of a raw material, cellulose acylate (DAC "L-50" produced by Daicel Corporation, cellulose diacetate, weight-average polymerization degree: 570) is added, and the resulting mixture is heated and stirred at a dissolving temperature of 60°C to completely dissolve the raw material in ethyl acetate.

The obtained solution is added to a dispersion containing 500 parts of pure water and 45 parts of calcium carbonate serving as a dispersant, and the resulting mixture is stirred with a high-speed emulsifier for 10 minutes while maintaining the emulsifying temperature of 60°C.

The obtained emulsion is added to 60 to 75°C hot water in an amount five times the emulsion, and the resulting mixture is stirred all night to obtain a dispersion in which cellulose acylate particles are deposited.

To the obtained dispersion, diluted hydrochloric acid is added to dissolve calcium carbonate. After filtering out the residue of the dispersion, the residue is dispersed in pure water again to obtain a cellulose acylate particle dispersion (solid concentration: 10%).

### Saponifying step

To 120 parts of the obtained cellulose acylate particle dispersion, 17.5 parts of a 20% aqueous sodium hydroxide solution is added, followed by stirring for 6 hours at a saponifying temperature of 30°C. Hydrochloric acid is added to the suspension after saponification to adjust the pH to 7, and then the obtained cellulose particles are filtered out and washed with acetone. Next, filtration and washing of the cellulose particles are repeated until the electrical conductivity of the filtrate is 10 µs/cm or less to thereby obtain porous cellulose particles.

### Examples 2 to 18 and Comparative Example 1

Cellulose particles are obtained as in Example 1 except that the following conditions are changed according to the "particle production conditions" indicated in Table:
Emulsifying temperature
Amount of poor solvent in the mixed solvent containing ethyl acetate and heptanol
Amount of dispersant
Amount of 20% aqueous sodium hydroxide solution (indicated as amount of NaOH)

### Comparative Examples 2 and 3

The following commercially available cellulose particles are respectively used as cellulose particles of Comparative Examples 2 and 3.
Comparative Example 2: "CELLULOBEADS USF-X" by DAITO KASEI KOGYO CO., LTD.
Comparative Example 3: "CELLUFLOW C-25" by JNC Corporation

### Evaluation

### Properties of particles

For the porous cellulose particles obtained in each of the examples, the following properties of the particles are measured according to the methods described above:
BET specific surface area
Area ratio of open cells
Degree of substitution of cellulose derivative
Volume-average particle size (in the table, also indicated as "Particle size") of cellulose particles

### Smoothness (rolling property)

The "smoothness" of the pressed porous cellulose particles of each of the examples felt when the pressed porous cellulose particles are applied to the skin is rated by 20 participants on a one-to-five scale. 5 indicates the highest smoothness feel on the skin. The average value of the one-to-five scale ratings given by 20 participants is converted into A+ to D ratings according to the following evaluation standard, where the ratings of A+ to C are judged as having excellent smoothness, and the rating of D is judged as having poor smoothness. A+ indicates the highest smoothness, and D indicates the lowest smoothness.

Here, in pressing the particles, 8 g of the particles are loaded onto a round metal plate (diameter: 5.5 cm) and pressed at a pressing pressure of 500 kgf/cm² for a pressing time of 10 seconds.
A+: The average value of the ratings is 4.5 or more.
A: The average value of the ratings is 4 or more and less than 4.5.
B: The average value of the ratings is 3.5 or more and less than 4.
C: The average value of the ratings is 3 or more and less than 3.5.
D: The average value of the ratings is less than 3.

### Sebum adsorbing property (oil absorbing property)

The linseed oil absorption rate of the porous cellulose particles of each of the examples after pressing is measured. Specific details are as follows.

Ten grams of linseed oil and 1 g of pressed porous cellulose particles of each example are mixed, and the resulting mixture is centrifuged at a rotation rate of 10,000 rpm for 30 minutes. The particle weight is measured after removing the supernatant linseed oil, and the oil absorbing property is calculated from the following equation. Oil absorption rate = (increase in weight after centrifugation ÷ weight of particles before centrifugation) × 100

The adsorbing property is evaluated according to the following standard.

Here, in pressing the particles, 8 g of the particles are loaded onto a round metal plate (diameter: 5.5 cm) and pressed at a pressing pressure of 500 kgf/cm² for a pressing time of 10 seconds.
A+: 250% ≤ oil absorption rate
A: 220% ≤ oil absorption rate < 250%
B: 190% ≤ oil absorption rate < 220%
C: 160% ≤ oil absorption rate < 190%
D: oil absorption rate < 160%

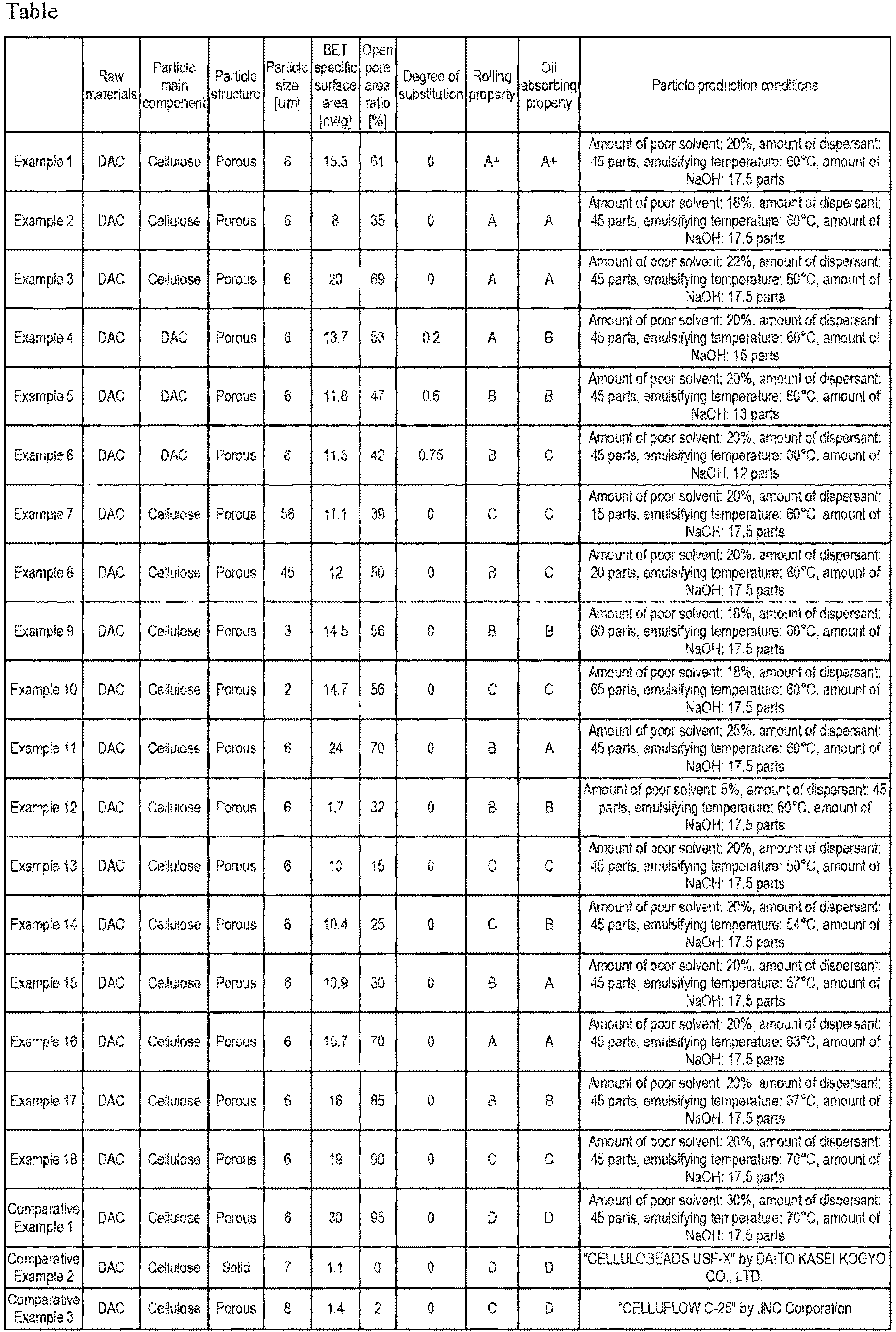

The aforementioned results indicate that the cellulose particles of Examples exhibit excellent mechanical strength and excellent smoothness and sebum absorbing properties even after application of mechanical load compared to the particles of Comparative Examples.

Thus, these results also indicate that the cellulose particles of Examples exhibit excellent mechanical strength and excellent rolling properties and oil absorbing properties even after application of mechanical load compared to the particles of Comparative Examples.

The foregoing description of the exemplary embodiments of the present disclosure has been provided for the purposes of illustration and description. It is not intended to be exhaustive or to limit the disclosure to the precise forms disclosed. Obviously, many modifications and variations will be apparent to practitioners skilled in the art. The embodiments were chosen and described in order to best explain the principles of the disclosure and its practical applications, thereby enabling others skilled in the art to understand the disclosure for various embodiments and with the various modifications as are suited to the particular use contemplated. It is intended that the scope of the disclosure be defined by the following claims and their equivalents.

### Appendix

(((1))) Porous cellulose particles comprising:
   a cellulose, a cellulose derivative, or a mixture thereof as a main component, wherein the porous cellulose particles have open cells extending from a particle surface to a particle interior, and have a BET specific surface area of 1.7 m²/g or more and 24.0 m²/g or less.
(((2))) The porous cellulose particles described in (((1))), wherein the BET specific surface area is 8.0 m²/g or more and 20.0 m²/g or less.
(((3))) The porous cellulose particles described in (((1))) or (((2))), wherein, when cross sections of the porous cellulose particles are observed, an area ratio of the open cells is 15% or more and 90% or less.
(((4))) The porous cellulose particles described in (((3))), wherein, when cross sections of the porous cellulose particles are observed, the area ratio of the open cells is 25% or more and 85% or less.
(((5))) The porous cellulose particles described in any one of (((1))) to (((4))), wherein the cellulose derivative is cellulose acylate having a degree of substitution of 0.6 or less.
(((6))) The porous cellulose particles described in (((5))), wherein the cellulose derivative is cellulose acylate having a degree of substitution of 0.2 or less.
(((7))) The porous cellulose particles described in any one of (((1))) to (((6))), wherein the porous cellulose particles have a volume-average particle size of 3 µm or more and 45 µm or less.
(((8))) A cosmetic preparation comprising the porous cellulose particles described in any one of (((1))) to (((7))).

According to (((1))), there is provided porous cellulose particles that contain a cellulose, a cellulose derivative, or a mixture thereof as a main component and that have open cells extending from a particle surface to a particle interior, according to which porous cellulose particles having excellent mechanical strength and excellent rolling property and oil absorbing property after application of mechanical load are provided compared to when the BET specific surface area is less than 1.7 m²/g or more than 24.0 m²/g.

According to (((2))), there is provided porous cellulose particles that have excellent mechanical strength and excellent rolling property and oil absorbing property after application of mechanical load compared to when the BET specific surface area is less than 8.9 m²/g or more than 20.0 m²/g.

According to (((3))), there is provided porous cellulose particles that have excellent mechanical strength and excellent rolling property and oil absorbing property after application of mechanical load compared to when the area ratio of the open cells is less than 15% or more than 90%.

According to (((4))), there is provided porous cellulose particles that have excellent mechanical strength and excellent rolling property and oil absorbing property after application of mechanical load compared to when the area ratio of the open cells is less than 25% or more than 85%.

According to (((5))), there is provided porous cellulose particles that have excellent mechanical strength and excellent rolling property and oil absorbing property after application of mechanical load compared to when the cellulose derivative is cellulose acylate having a degree of substitution exceeding 0.6.

According to (((6))), there is provided porous cellulose particles that have excellent mechanical strength and excellent rolling property and oil absorbing property after application of mechanical load compared to when the cellulose derivative is cellulose acylate having a degree of substitution exceeding 0.2.

According to (((7))), there is provided porous cellulose particles that have excellent mechanical strength and excellent rolling property and oil absorbing property after application of mechanical load compared to when the porous cellulose particles have a volume-average particle size of less than 3 µm or more than 45 µm.

According to (((8))), there is provided a cosmetic preparation that has excellent mechanical strength and excellent smoothness and sebum adsorbing property after application of mechanical load compared to when porous cellulose particles that contain a cellulose, a cellulose derivative, or a mixture thereof as a main component, that have open cells extending from a particle surface to a particle interior, and that have a BET specific surface area of less than 1.7 m²/g or more than 24.0 m²/g are used.

## Claims

1. Porous cellulose particles comprising:
a cellulose, a cellulose derivative, or a mixture thereof as a main component, wherein the porous cellulose particles have open cells extending from a particle surface to a particle interior, and have a BET specific surface area of 1.7 m²/g or more and 24.0 m²/g or less.

2. The porous cellulose particles according to claim 1, wherein the BET specific surface area is 8.0 m²/g or more and 20.0 m²/g or less.

3. The porous cellulose particles according to claim 1 or 2, wherein, when cross sections of the porous cellulose particles are observed, an area ratio of the open cells is 15% or more and 90% or less.

4. The porous cellulose particles according to claim 3, wherein, when cross sections of the porous cellulose particles are observed, the area ratio of the open cells is 25% or more and 85% or less.

5. The porous cellulose particles according to any one of claims 1 to 4, wherein the cellulose derivative is cellulose acylate having a degree of substitution of 0.6 or less.

6. The porous cellulose particles according to claim 5, wherein the cellulose derivative is cellulose acylate having a degree of substitution of 0.2 or less.

7. The porous cellulose particles according to any one of claims 1 to 6, wherein the porous cellulose particles have a volume-average particle size of 3 µm or more and 45 µm or less.

8. A cosmetic preparation comprising the porous cellulose particles according to any one of claims 1 to 7.
